# EUROPEAN PATENT APPLICATION

(11) **EP 4 678 127 A1**
(43) Date of publication of application: **14.01.2026**
(21) Application number: 25188308.8
(22) Date of filing: 09.07.2025
(51) Int. Cl.: A61B 18/14, A61B 18/00

(54) **COLLAPSIBLE PLANAR CATHETER**

(30) Priority: 10.07.2024 US 202463669318 P; 10.06.2025 US 202519233101
(71) Applicant: Biosense Webster (Israel) Ltd., 2066717 Yokneam (IL)
(72) Inventor: VAN NIEKERK, Pieter Emmelius, Irvine, 92618 (US); HIGHSMITH, Debby, Irvine, 92618 (US); RODRIGUEZ SOTO, Juan, Irvine, 92618 (US); EBRAHIMI, Babak, Irvine, 92618 (US); ABBAS, Mohammad, Irvine, 92618 (US); PEDROSO, Pedro D., Raynham, 02767 (US)
(74) Representative: Carpmaels & Ransford LLP

(57) **Abstract**

A medical probe including an end effector is herein disclosed. The end effector includes a flexible insulative material extending along a plane, a framework disposed in the flexible insulative material, and a flexible circuit disposed in the flexible insulative material and spaced apart from the framework. The flexible circuit includes a central branch segment, a plurality of outer branch segments, and a plurality of electrodes. The central branch segment extends along a longitudinal axis of the plane. The outer branch segments extend along the plane a distance away from the longitudinal axis, with at least some of the outer branch segments extending distal to the central branch segment thereby defining a recess in a distal end of the end effector. The electrodes are disposed along the central branch and the plurality of outer branch segments.

## Description

### CROSS-REFERENCE TO RELATED APPLICATIONS

The application claims benefit of priority to prior filed U.S. Provisional Patent Application No. 63/669,318 filed July 10, 2024 (Attorney Docket No.: BIO6934USPSP1 - 253757.000495), which is hereby incorporated by reference in full herein.

### FIELD

The present disclosure relates generally to minimally invasive medical devices, and in particular cardiac mapping and/or ablation catheters with flexible probe tips.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within short time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible probe tips designed to overcome this disadvantage. These catheters can include layered components that can be time-consuming, complex, and expensive to manufacture and assemble. Moreover, electrical traces and other components associated therewith can be prone to breakage and/or delamination when in use.

### SUMMARY

There is provided, in accordance with the disclosed technology, an end effector. The end effector includes a flexible insulative material extending along a plane, a framework disposed in the flexible insulative material, and a flexible circuit disposed in the flexible insulative material and spaced apart from the framework. The flexible circuit includes a central branch segment, a plurality of outer branch segments, and a plurality of electrodes. The central branch segment extends along a longitudinal axis of the plane. The outer branch segments extend along the plane a distance away from the longitudinal axis, with at least some of the outer branch segments extending distal to the central branch segment thereby defining a recess in a distal end of the end effector proximate the longitudinal axis. The electrodes are disposed along the central branch and the plurality of outer branch segments.

There is provided, in accordance with the disclosed technology, an end effector. The end effector includes a flexible insulative material extending along a plane, a framework disposed in the flexible insulative material, a first flexible circuit disposed in the flexible insulative material and spaced apart from the framework on one side of the framework, and a second flexible circuit disposed in the flexible insulative material and spaced apart from both the framework and the first flexible circuit. Each of the first and second flexible circuits include a central branch segment extending along a longitudinal axis of the plane, a first set of electrodes disposed on the central branch segment, a plurality of outer branch segments extending along the plane a distance away from the longitudinal axis, and a second set of electrodes disposed along adjacent outer branch segments, with the first set of electrodes and the second set of electrodes being unaligned with each other in a direction transverse to the longitudinal axis.

There is provided, in accordance with the disclosed technology, a method of manufacturing an end effector for a medical probe. The method includes forming an electrode on a flexible circuit, coating the electrode with an impedance reducing coating, and coating the electrode with a hydrophilic coating.

There is provided, in accordance with the disclosed technology, a method of manufacturing a flexible circuit for a medical probe. The method includes forming a substrate extending along a plane, disposing a conductive material along the substrate, and cutting through the conductive material and the substrate along a curve of the flexible substrate such that the flexible substrate and the conductive material form a plurality of individual branches.

### BRIEF DESCRIPTION OF THE DRAWINGS

FIG. 1 is a schematic pictorial illustration of a medical system including a planar medical probe, in accordance with the disclosed technology;
FIG. 2A is a schematic pictorial illustration of a plan view of a probe tip, in accordance with the disclosed technology;
FIG. 2B is a schematic pictorial illustration of a plan view of another probe tip, in accordance with the disclosed technology;
FIG. 3 is a schematic pictorial illustration of an exploded perspective view of the probe tip of FIG. 2A;
FIG. 4 is a schematic pictorial illustration of a plan view of a probe tip, in accordance with the disclosed technology;
FIG. 5A is a schematic pictorial illustration of a probe tip collapsed within a sheath in an idealized case, in accordance with the disclosed technology;
FIG. 5B is a schematic pictorial illustration of a probe tip collapsed within a sheath in an exemplary case where excess material is present, in accordance with the disclosed technology;
FIG. 6A is a schematic pictorial illustration of a plan view of another probe tip, with certain portions thereof removed for clarity, in accordance with the disclosed technology;
FIG. 6B is a schematic pictorial illustration of a plan view of a position sensor, in accordance with the disclosed technology;
FIG. 7A is a schematic pictorial illustration of a plan view of a distal portion of a probe tip, in accordance with the disclosed technology;
FIG. 7B is a schematic pictorial illustration of a detail view of a typical flexible circuit of a probe tip, including a detail cross-sectional view of a section thereof;
FIG. 7C is a schematic pictorial illustration of a detail view of a portion of flexible circuit of a probe tip, including a detail cross-sectional view of a section thereof, in accordance with the disclosed technology;
FIG. 7D is a schematic pictorial illustration of a detail view of another flexible circuit of a probe tip, in accordance with the disclosed technology;
FIG. 7E is a schematic pictorial illustration of a detail view of another flexible circuit of a probe tip, in accordance with the disclosed technology;
FIG. 8 is a flow diagram of a method of making a flexible circuit with one or more strain relief loops, in accordance with the disclosed technology;
FIG. 9A is a schematic pictorial illustration of a detail view of an electrode provided with an impedance reducing coating, in accordance with the disclosed technology;
FIG. 9B is a schematic pictorial illustration of a detail view of a coated electrode, in accordance with the disclosed technology;
FIG. 10 is a schematic pictorial illustration of a graph of resistance over a range of frequencies when lubricant is used and when no lubricant is used, in accordance with the disclosed technology;
FIG. 11A is a schematic pictorial illustration of a multi-variable chart for peak-to-peak data of coated and uncoated electrodes, in accordance with the disclosed technology;
FIG. 11B is a schematic pictorial illustration of a multi-variable chart of impedance at 10 Hz of coated and uncoated electrodes, in accordance with the disclosed technology; and
FIG. 12 is a flow diagram of a method of making a flexible circuit with a coated electrode, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

The present disclosure is related to systems, methods, uses, and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

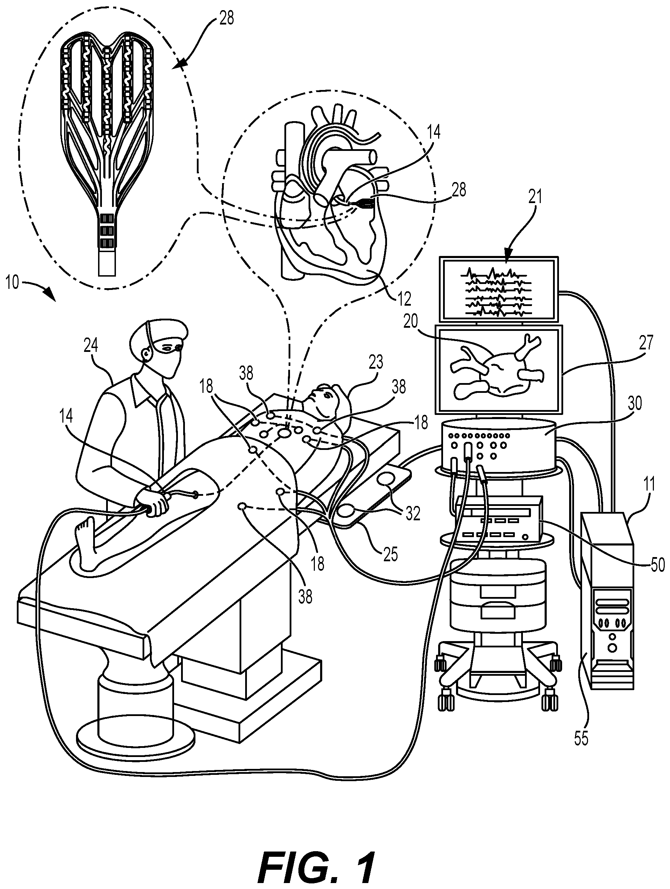

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter/medical probe 14 that is configured for sensing IEGM is illustrated herein. Physician 24 brings a catheter shaft with distal tip of catheter 14 (i.e., multilayered probe tip 100) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 102 optionally distributed over probe tip 28 coupled to a catheter shaft and configured to sense the IEGM signals as described in more detail below. Catheter 14 may additionally include a position sensor embedded in or near probe tip 28 for tracking position and orientation of probe tip 28. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of probe tip 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 102. For impedance-based tracking, electrical current is directed toward electrodes 102 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 102 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 102 at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

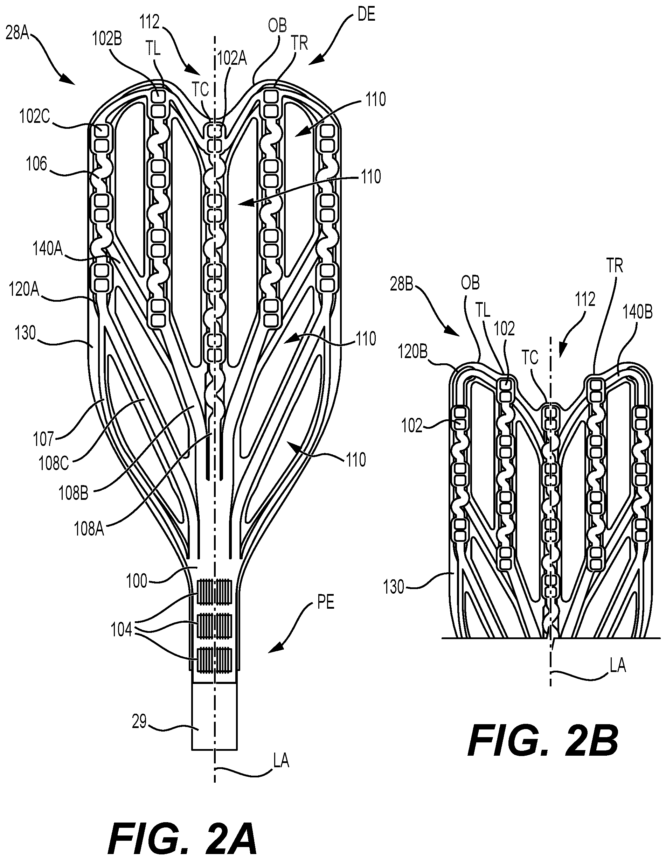

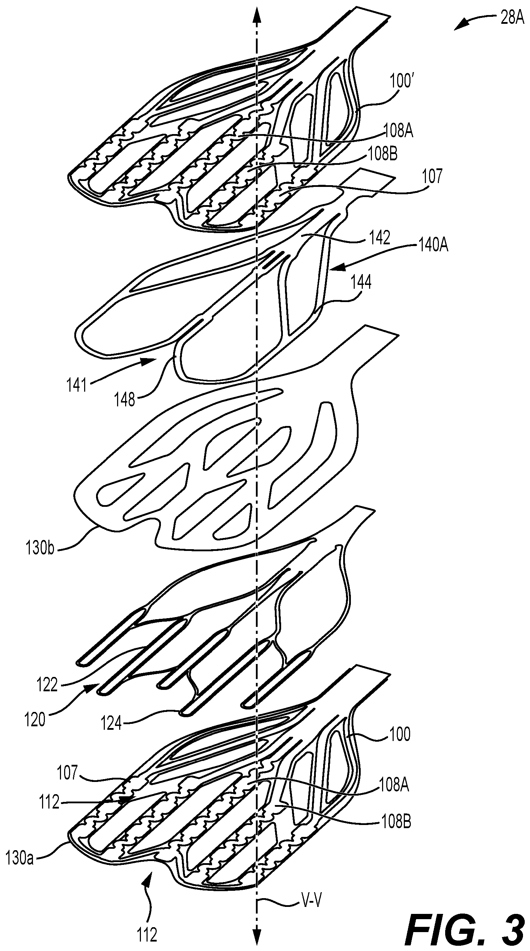

FIGs. 2A and 2B provide views of two exemplary probe tips 28A, 28B (also referred to herein as an end effector) that are configured for insertion into an internal body cavity of a patient, while FIG. 3 shows an exploded view of the probe tip 28A, with the components thereof exploded vertically along vertical axis V-V.

Making reference to FIGs. 2A and 3, the probe tip 28A extends from a proximal end PE (that connects to an elongated shaft 29) to a distal end DE along a longitudinal axis LA and includes a first flexible circuit 100 including a plurality of electrodes 102, as well as a second flexible circuit 100' that is identically configured as the first flexible circuit 100. In some examples, the term "flexible circuit" includes thin-film circuit, flexible printed circuit board, thin film deposition via lithography and etching processes on substrates such as polyimide, copper, LCP, nitinol substrate, thermoplastic polyurethane (TPU), silicone, thermoset resin, or other polymeric substrates. In some examples, the flexible circuits described herein can be made primarily of polyimide. In other examples, it can be made of any of biocompatible polyimides, glass-reinforced epoxy laminate materials, copper, or graphene, alone or in combination. In some examples, the electrodes 102 described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The flexible circuits 100, 100' are disposed in an insulative material 130 that extends along the longitudinal axis LA of the plane. The insulative material 130 can be contiguous to the contact surfaces of the electrodes 102 so that only the contact surfaces of at least a portion of the plurality of electrodes 102 are exposed to the ambient environment. As used herein, "ambient environment" refers to the external environment such as the organ in which the probe tip 100 is deployed or in the operating theater prior to being deployed in the biological organ. The insulative material 130 at least partially encapsulates and spaces the different layers of the probe tip 28A (e.g., the flexible circuits 100, 100' and the framework 120, which is discussed in greater detail below) along a vertical axis V-V.

A flexible substrate of each flexible circuit 100, 100' comprises a bio-compatible material and extends along the plane and has a first side and a second side. In some examples, the flexible substrate is formed entirely or about entirely from the bio-compatible material. The electrodes 102 are disposed on a surface of the flexible substrate. The electrodes 102 are disposed on only one side of the plane. Put another way, the electrodes 102 are directed so as to face away from the framework 120.

With specific reference to FIG. 2A, each flexible circuit 100, 100' includes a plurality of electrodes 102, a soldering pad region 104 disposed at a proximal end thereof, a central branch segment 108A, a plurality of outer branch segments 108B, 108C, and a plurality of voids 110 defined between the branch segments 108A-108C. The central branch segments 108A extend along the longitudinal axis LA of the plane. The electrodes 102 can include respective sets 102A, 102B, 102C disposed along the central branch 108A (e.g., electrodes 102A) and the outer branch segments 108B (e.g., electrodes 102B, 102C) that are staggered along the longitudinal axis LA. As seen, for example, in FIG. 2A, one or more of the segments of the flexible circuits 100, 100' can include a winding serpentine pattern 106. Additionally, a connecting outer segment 107 can be provided, that does not include electrodes 102, to aid in defining the shape of the distal tip 28 and provide reinforcement/protection to the segments of the flexible circuit 100 that carry electrical traces and/or electrodes. The outer branch segments 108B, 108C extend along the plane a distance away from the longitudinal axis LA. At least some of the outer branch segments 108B, 108C extend distal to the central branch segment 108A, thereby defining a recess 112 in a distal end of the end effector 28A.

As used herein, the recess 112 is defined by the outer boundary OB (i.e., a perimeter) of the end effector converging or receding towards the longitudinal axis LA such that the outer boundary OB of a terminal portion of the flexible circuit 100 or 100' (and also the end effector 28 as a whole) proximate the longitudinal axis LA (also referred to herein as a terminal central portion TC) is recessed at least with respect to the outer boundaries of the nearest adjacent distal terminal portions TR or TL of the flexible circuit 100 or 100', as exemplarily seen in FIGs. 2A and 2B. While the preferred examples have the outer boundary OB of the insulative material 130 follow the outer perimeter of the flexible circuit 100 or 100' to define the recess 112, it is noted that at a minimum, the outer perimeter of the flexible circuit 100 or 100' does not extend beyond the outer perimeter of the insulative material 130 to ensure that the sharp edges of the flexible circuit 100 or 100' do not intrude into body tissues.

It is noted that not all of the electrodes 102A-102C on the probe tip 28 described herein need be exposed through the insulative material 130 as these non-exposed electrodes can be used to sense far-field signals for noise reduction proximate the tissue contacting electrodes. Similarly, far-field signals including noise or artifacts can be reduced or canceled out for the overall probe tip with a reference electrode that is not in contact with tissues and only with blood.

The probe tip 28A can further include a framework 120A contiguous to the insulative material 130 or in the insulative material 130. In examples in which the probe tip 28 includes framework 120A, the framework 120A can be disposed directly on the flexible circuit 100 with none, or very little, of the insulative material 130 coming between the two. In other examples, insulative layers of the insulative material 130 can space the framework 120A from the flexible circuits 100, 100'.

In some examples, the framework 120A is disposed in the insulative material 130 and is substantially planar along the longitudinal axis LA such that the longitudinal axis LA is parallel to or coincident with the framework 120A. In the example shown in FIG. 2, a position sensor 140A can be provided that is sandwiched between the flexible circuit 100 and the spine framework 120A and generally parallel thereto. In some examples, the framework 120A is formed from a flexible, resilient material. By way of example, the framework can be formed from a shape-memory alloy such as nickel-titanium, also known as Nitinol, cobalt chromium, stainless steel, and/or other alloys that exhibit pseudo-elastic and/or super-elastic properties.

Stated otherwise, an aspect of the present disclosure provides a probe tip 28 having a planar framework 120A bisecting two flat, heat formed portions of a flexible insulating mass 130, with at least one flexible circuit 100 (and/or flexible circuit 100') disposed on one side of the framework 120A.

Framework 120A can be a component of the probe tip 28 that is separate and distinct from the first flexible circuit 100 and disposed proximate the first flexible circuit 100. In this case, the insulative material 130 can be further disposed between the framework 120A and the second flexible circuit 100'. The framework 120A can be formed from a planar or cylindrical stock of material using any suitable method. For example, the framework 120 can be formed by cutting, laser cutting, stamping, etc.

Insulative material 130 can include one or more sheets fused together proximate the framework 120 into a single, contiguous, generally planar insulative mass 130. This insulative material 130 also serves to enhance the atraumaticity of the probe tip 100 and to protect the subject from sharp edges. The insulative material 130 can include polymer. The insulative material 130 can be heat formed around at least a portion of the first flexible circuit 110, the second flexible circuit 100', and the framework 120A. The polymer can include TPU or other heat formed or shaped material which lends itself to said heat forming.

Furthermore, while the insulative material 130 is shown to be flat in these figures, insulative material 130 can be shaped, scalloped, ribbed, ridged, concaved, convexed, or otherwise configured such that the overall profile of insulative material 130 yields physical and/or mechanical properties, such as rigidity and flexion along multiple axes, required by the probe tip 28, mentioned above.

FIG. 2B illustrates an alternative configuration of the distal tip 28B with a different geometry than that of the distal tip 28A of FIG. 2A, but that still defines a recess 112 by virtue of at least some of its outer branch segments, its spine framework 120B, and its position sensor 140B extending further along the longitudinal axis LA than the central branch segment.

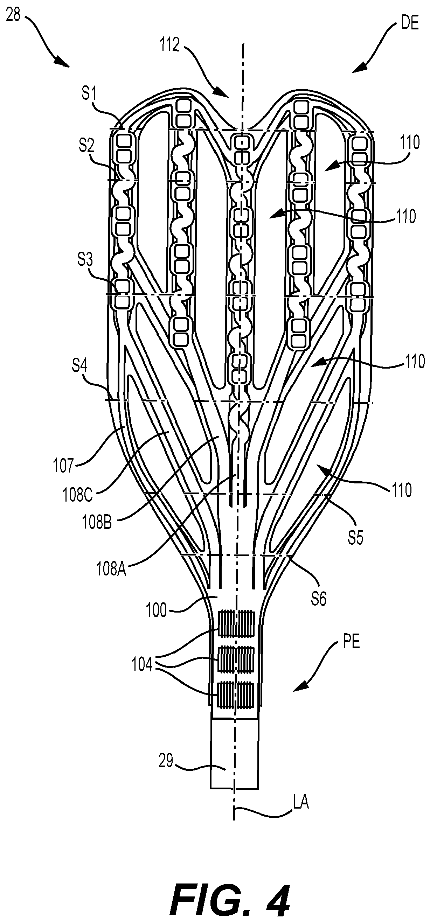

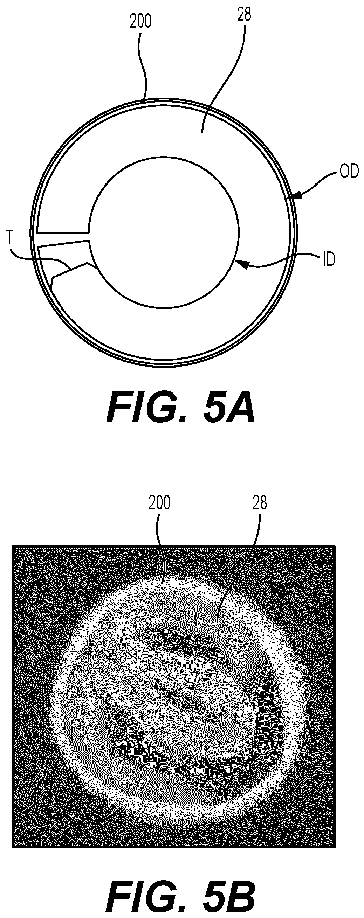

Turning now to FIGs. 4-5B, further features of the disclosed technology are shown in greater detail. In use, when the distal tip 28 (e.g., the distal tip 28A previously described) is retracted into an insertion tool 200 (FIGs. 5A-5B), it needs to deform and/or roll such that all the material fits within the prescribed circumference (e.g., see FIGs. 5A and 5B). Because of that, as demonstrated particularly in FIG. 5A, a thickness T of the distal tip 28 directly correlates with a resultant inner diameter ID of the distal tip 28 when it collapses within the insertion tool 200. If the inner diameter ID is too small, the width of the distal tip 28 will not be provide sufficient coverage in a clinical setting. In practical use and, in particular, in cases where excess material is used (discussed more in the next paragraph), the distal tip 28 doesn't collapse perfectly cylindrically, but rather collapses on itself to fill the inner space of the insertion tool 200, such as the orientation shown in FIG. 5B. Nevertheless, if the inner diameter ID is too large, the distal tip 28 may not predictably collapse and its collapsing can cause strain on and/or failure to the sub-components (e.g., the flexible circuit 100).

Therefore, the presently disclosed distal tip 28, as shown in FIG. 4, employs a combination of material voids 110 between the segments, where electrodes 102 are not needed/used, and a recess 112 at the distal end DE. Along the longitudinal axis LA, at any given cross-section of the distal tip 28 cut perpendicular to the longitudinal axis 28 (e.g., sections S1, S2, S3, S4, S5, and S6), each section S1, S2, S3, S4, S5, S6 has a predetermined maximum total width of material based on the desired access sheath French size compatibility of the distal tip (i.e., the voids 110 and recess 112 do not count towards that width). Put another way, a sum of the width of the central branch segment and the plurality of outer branch segments of the distal tip 28 and of the segments 108A-108C of the flexible circuit 100 in a direction transverse to the longitudinal axis LA is less than a threshold width at any given location along the distal tip 28. In other words, a linear cross-sectional measurement of the distal tip 28A is minimized at all locations along the longitudinal axis LA. In the presently described example, the maximum total width of material at any given cross-section of the distal tip 28 is as close as possible to *π(d-2T),* with *d* being the inner diameter of the desired access sheath/insertion tool 200, and T being the thickness of the distal tip 28. This enables the distal tip 28 to collapse similarly to the depiction shown in FIG. 5A. Any width in excess of this will have to collapse inwardly, as indicated in FIG. 5B. By way of non-limiting example, if the sheath inner diameter is 2.9mm, and the distal tip thickness is 0.4mm, the maximum material at any cross section should be less than 6.6mm (with a total width of the distal tip 28 being approximately 11mm to 12mm).

To at least partially define these voids 110, the outer branch segments 108B, 108C extend outwardly from the central branch segment 108A at an oblique angle with respect to the central branch segment 108A and the longitudinal axis LA for at least a portion of the outer branch segments 108B, 108C. More specifically, a proximal portion (see the lower portion in, e.g., FIG. 4) of each outer branch segment 108B, 108C extends outwardly from the central branch segment 108A at an oblique angle with respect to the longitudinal axis LA and a distal portion (see the upper portion in, e.g., FIG. 4) of the outer branch segments 108B, 108C extend parallel to the central branch segment 108A, 108B.

As seen for example in FIGs. 2A and 4, the electrodes 102 are staggered, which enables the distal end DE to be more easily configured to define the recess 112 (with only certain electrodes 102B, as seen in FIG. 2A, being disposed at the distal end DE). More specifically, the plurality of electrodes include a first set 102A of electrodes 102 disposed on the central branch segment 108A and a second set 102B of electrodes 102 disposed on/along an adjacent branch 108B of the plurality of outer branch segments 108B, 108C. The first set 102A of electrodes 102 and the second set 102B of electrodes 102 are unaligned (i.e., staggered) with each other in a direction transverse to the longitudinal axis LA. With this configuration, the insulative material 130 has an undulating pattern in the direction transverse to the longitudinal axis LA, thereby reducing the total amount of material at any given linear cross-sectional measurement.

Further to the above, the outer branch segments 108B, 108C can further include a first set 108B of outer branch segments and a second set 108C of outer branch segments (see the symmetrical left and right side of the distal tip 28A, the respective sets being formed by one respective segment on the left side and one respective segment on the right side). The first set 108B of outer branch segments extend distal to the central branch segment 108A and the second set 108C of outer branch segments terminate at a location approximately aligned with the central branch segment 108A in a direction transverse to the longitudinal axis LA, with a bridge segment connecting the distalmost ends of the segments 108A, 108B, 108C. With this configuration, the electrodes 102C disposed along the second set 108C of outer branch segments align with electrodes 102A disposed along the central branch segment 108A.

It is noted that the alternative distal tip 28B employs a similar configuration of flexible circuit 110, with the primary difference being how the other layers (e.g., framework 120B and position sensor 140B) are configured, while still maintaining the aforementioned minimized linear cross-sectional measurement of the distal tip 28B (excluding the voids 110 and recess 112).

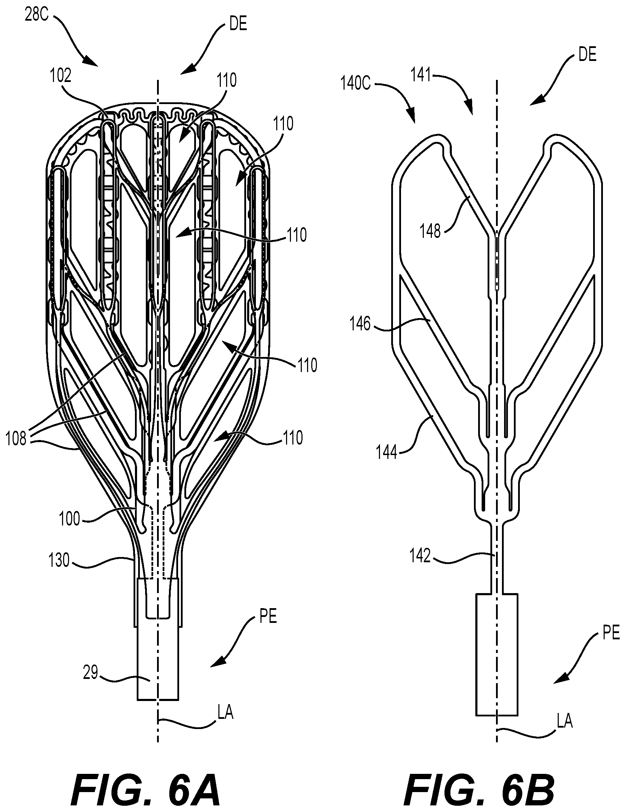

Turning now to FIGs. 6A-6B, an alternative configuration 28C of the distal tip 28 includes the aforementioned voids 110 and also includes a distal end DE shaped be less than a threshold width at any given location along the end effector 28 (like the previously described examples. The distal tip 28C includes a position sensor 140C extending from a proximal end PE to a distal end DE along the longitudinal axis LA. The position sensor includes a center segment 142 extending along the longitudinal axis and a plurality of outer segments 144, 146, 148 that extend from the center segment 142 at an oblique angle. The distalmost outer segments 148 together define a recess 141 which aids in reducing strain when the distal tip 28C is rolled into the insertion tool 200 or another delivery sheath. Moreover, the electrical traces on the position sensor 140C exit in a direction parallel to the central segment 142. With the presently shown/described design, the primary folding direction of the distal tip 28 is perpendicular to the plane of the position sensor 140C, and will largely be concentrated at the proximal end of the distal tip 28. The traces are parallel to the centerline in the present example to take advantage of rolled annealed copper cladding (where the copper grains are large in one direction). It is also noted that the center segment 142 is also narrow, which helps to reduce the torsional contribution of the position sensor 140C in this area.

In this example, the position sensor 140C has one or more loops with the traces that, when subjected to a magnetic field, induce a current indicative of a position of the probe tip 28C. In the present example, and with reference to FIG. 6B, the position sensor 140C can include three loops: a first loop that makes a loop on the outer segments 144, 148 and the center segment 142 on the left side of the longitudinal axis LA, a second loop that makes a loop on the outer segments 144, 148 and the center segment 142 on the right side of the longitudinal axis LA, and one that overlaps, and a third loop that makes a loop on the outer segments 146, 148 and the center segment 142 on both sides of the longitudinal axis LA such that it partially overlaps distal regions of the first loop and the second loop.

It is noted that, referring back to FIG. 3, the position sensor 140 in other examples can take a similar form/function as that of the one described with respect to FIGs. 6A-6B, and, while shaped slightly differently, includes the same or similar segments 142, 144, 146, 148 and recess 141. In this example, the recess 141 at least partially aligns with the recess 112 in the distal end DE of the probe tip 28A.

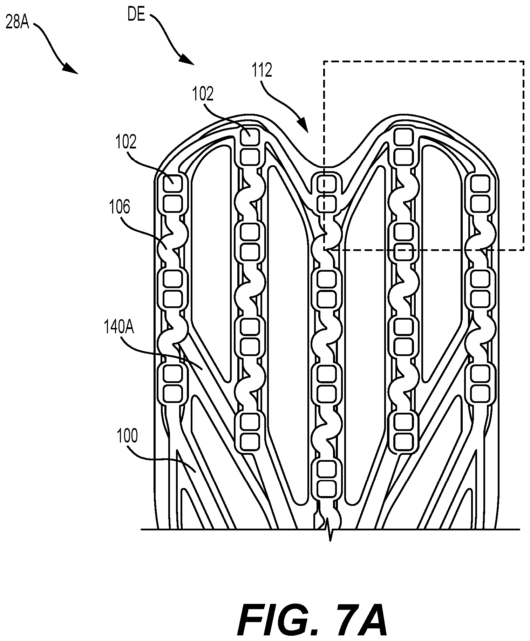

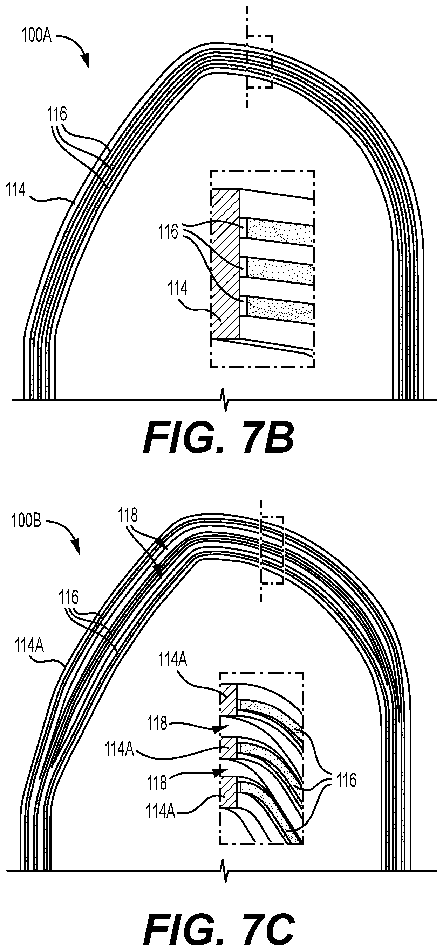

Reference is now made to FIGs. 7A-7E, which depict another aspect of the presently disclosed technology. With specific reference to FIG. 7A, a distal portion of the distal tip 28 of FIG. 2A is shown, with examples of the flexible substrate of the flexible circuit 100 bound by the dashed box enlarged in subsequent FIGs. 7B-7E.

Typical flexible circuits have a high aspect ratio, which results in a high preference for bending out of plane (i.e., along its weak axis) versus in plane (i.e., along one of its strong axes). As a result, typical flexible circuits undergo high compressive and tensile stress/strain when subjected to bending in plane, as well as torsion strain. One such flexible circuit is the flexible circuit 100A shown in FIG. 7B that includes a substrate 114 and one or more traces 116. FIG. 7B includes a detail cross-sectional view of the portion of the flexible circuit 100A bounded in the dashed line box to highlight its differences with the following example described with respect to FIG. 7C.

To address the stress/strain issues while bending, and as seen in FIG. 7C, a flexible circuit 100B that enables free movement about all three axes can be provided with any of the aforementioned examples of distal tip 28A. In this example, the traces 116 extend along the flexible circuit 100B and a substrate 114 supports each trace 116. Rather than providing all the traces 116 on one substrate 114 (as in FIG. 7B), the traces 116 are singulated on at least a portion (e.g., individual sections and the entire flexible circuit 100B can be singulated, as needed) of the flexible substrate 114 such that the flexible substrate 114 has a plural individual beams 114A that each correspond to and support a respective trace 116. The singulated trace 116 and beam 114A pairs define a plurality of gaps 118 between adjacent traces 116. With this configuration, the aspect ratio of the flexible circuit 100B can be greatly reduced, which increases their bendability along weak and strong axes while also torquing more easily.

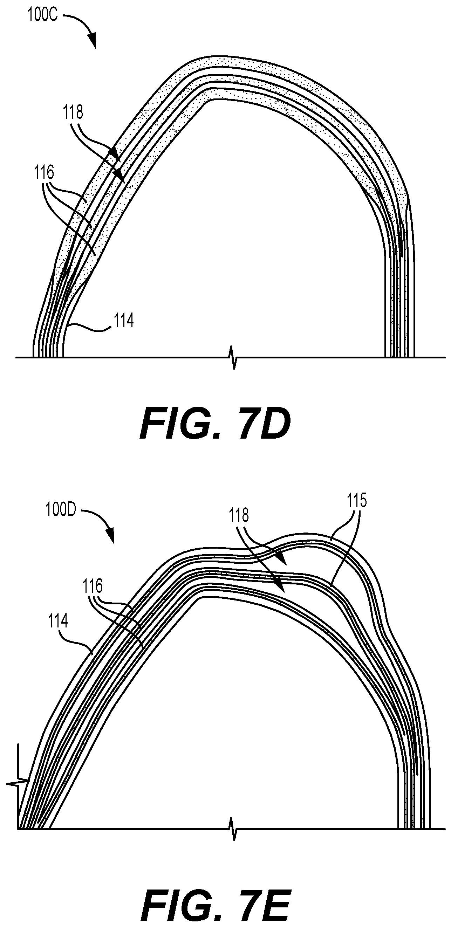

In the example shown in FIG. 7C, the flexible circuit 100B is cut between pre-etched traces 116. However, alternative configurations may be employed. FIG. 7D depicts a portion of another flexible circuit 100C that creates traces 116 by cutting directly through metal, thereby defining gaps 118 between the traces. Additionally, as seen in FIG. 7E with flexible circuit 100D, the substrate 114 and at least some of the traces 116 can further define strain relief loops 115 that promote in-plane bending by defining wider gaps 118 between adjacent strain relief loops 115 than that of the gaps 118 between other sections of the flexible circuit 100D.

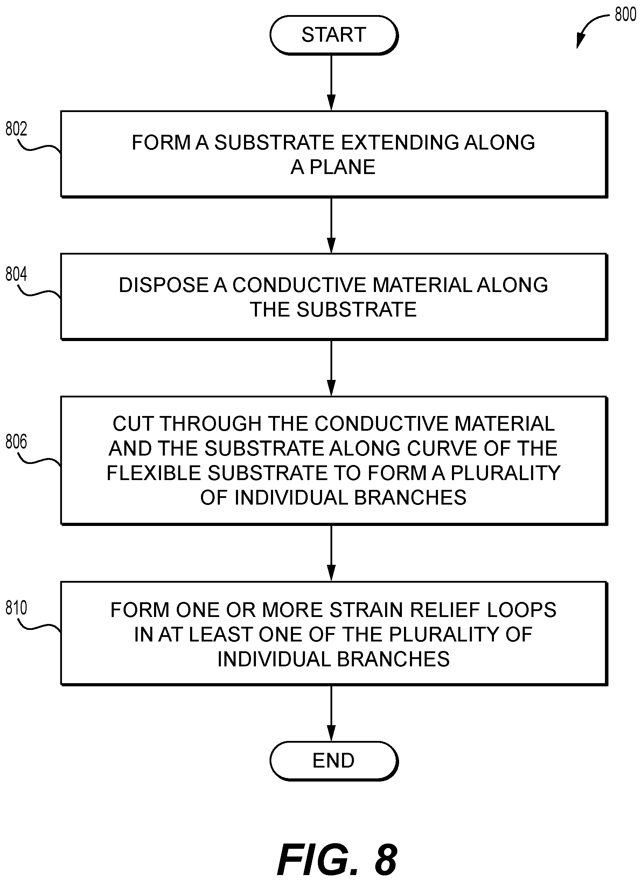

Further to the above, FIG. 8 illustrates a flow diagram of a method 800 of manufacturing a flexible circuit 100B, 100C for a medical probe 14, in accordance with the disclosed technology. A substrate is formed 802 such that it extends along a plane. A conductive material is disposed 804 along the substrate. In some examples, disposing 804 the conductive material along the substrate includes disposing individual traces of the conductive material along the substrate (e.g., as in the in the example of FIG. 7C where the traces 116 are pre-etched on the substrate 114). In some examples, disposing 804 the conductive material along the substrate includes disposing a single continuous portion of conductive material along the substrate (e.g., as in the example of FIG. 7D where the traces 116 are formed from a single piece of metal). The conductive material and the substrate are cut 806 through along a curve of the flexible substrate such that the flexible substrate and the conductive material form a plurality of individual branches (with gaps therebetween). In some examples, cutting 806 through the conductive material and the substrate includes cutting 806 through the conductive material and the substrate with a laser. In some examples, strain relief loops are formed 810 in at least one of the plurality of individual branches.

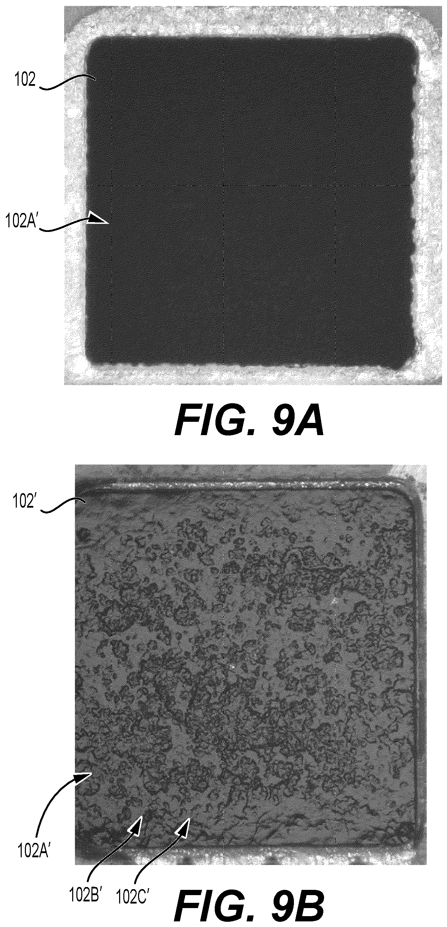

Reference is now made to FIGs. 9A-12 in conjunction with one another. The planar distal tip 28 described herein, in use, needs to have a low coefficient of friction during insertion of the tip 28 through the insertion tool 200 into a patient 23. Moreover, it is important that undesired information (i.e., noise) does not prevent proper function of the electrodes 102. Therefore, the present disclosure includes a method 1200 of manufacturing an end effector 28 for a medical probe 14 that includes coating an electrode 102 (as shown in FIG. 9A) with a impedance reducing coating 102A' (as shown in FIG. 9A) to improve the signal to noise ratio of the electrodes 102 and a hydrophilic coating 102B' (FIG. 9B) to reduce friction (i.e., the coating 102B' is lubricious) and to protect the electrodes 102 from contamination, with the final end effector 28 including protected coated electrodes 102' with a uniform lubricity over the entirety of the end effector 28. With the coating(s), there is a textured finish and a satin appears to the electrodes 102' (compared with a matte appearance when it uncoated). It is noted that the hydrophilic coating 102B' can also mechanically protect the impedance reducing coating 102A' even without being on top (fully or partially) on the noise-reducing coating due to the lubricious properties of the hydrophilic coating 102B' (which can be, e.g., hydrogel-based), thus allowing for a reduced friction action. Put another way, in use, the hydrophilic coating 102B' allows the distal tip 28 to slide easier such that it does not catch on anything that could harm the impedance reducing coating 102A'.

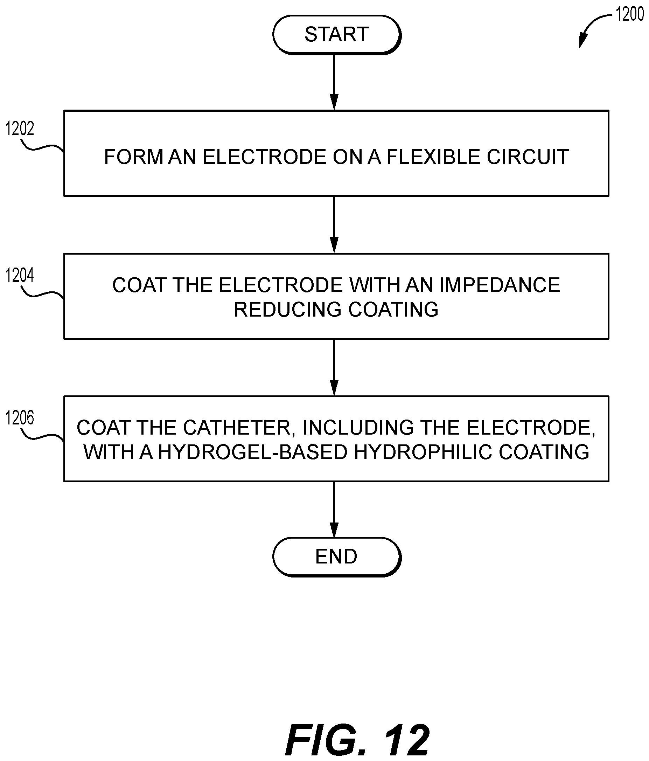

Making reference to FIG. 12, the method 1200 can include the following. An electrode 102 is formed 1202 on a flexible circuit 100. The electrode 102 is coated 1204 with an impedance reducing coating 102A'. The distal tip 28, including the electrode 102', is further coated 1206 with a hydrogel-based hydrophilic coating 102B' disposed at least partially over the impedance reducing coating 102A', resulting in a coated electrode 102'. Moreover, it is noted that when the hydrogel-based hydrophilic coating 102B' is applied immediately after the impedance reducing coating 102A', it allows the impedance reducing coating 102A' to stay hydrated, and no hydration and/or activation is needed prior to use. In some examples, the hydrophilic coating includes a hydrogel (e.g. LUBRICENT^{™}, available from Harland Medical Systems as of the filing date of the present application). In some examples, a majority or all of the distal tip 28, including the coated electrode 102', is optionally further coated with another lubricious coating 102C' (FIG. 9B).

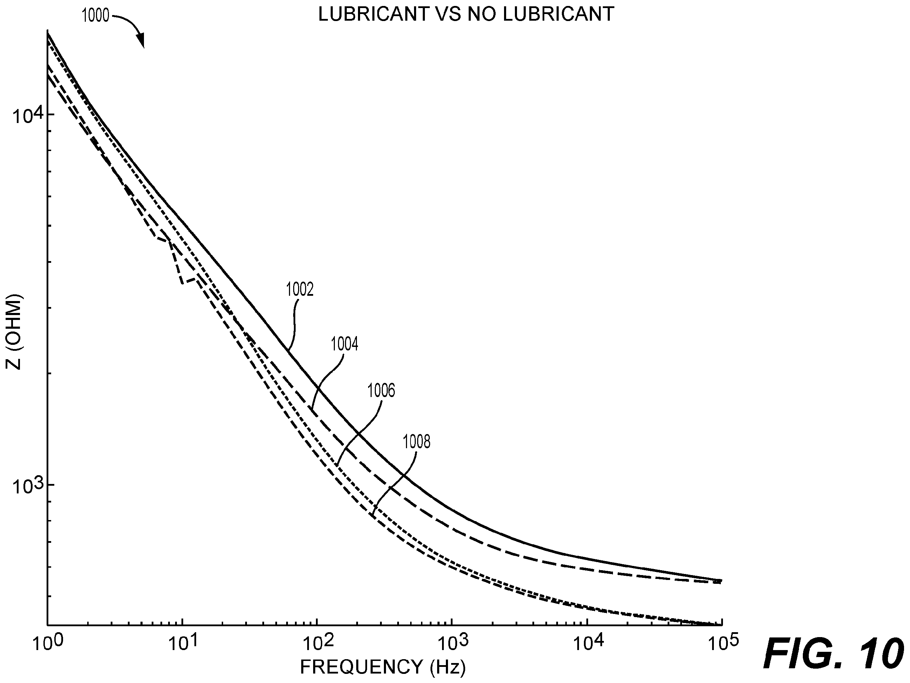

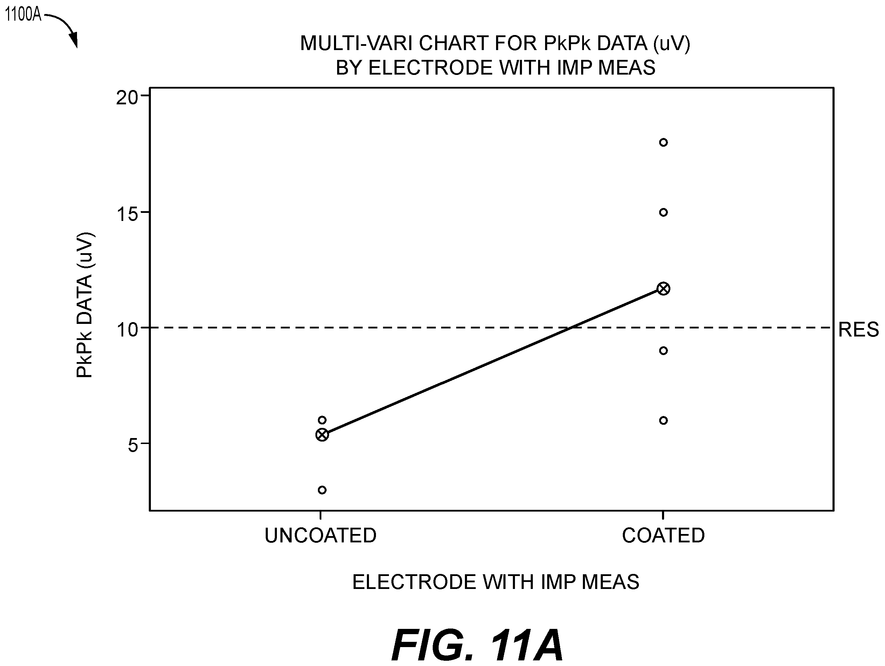

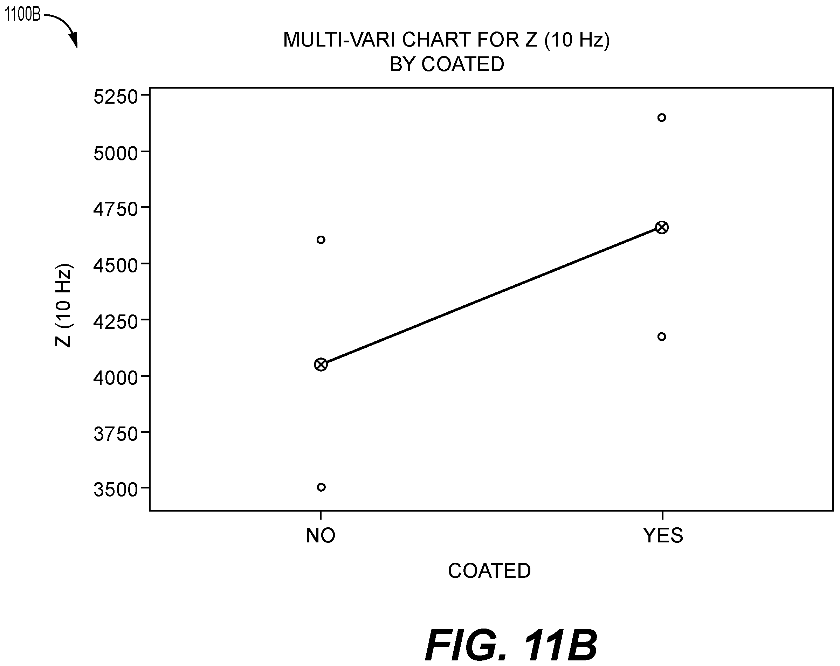

FIG. 10 is a graphical depiction 1000 of electrode resistance measured over a range of frequencies. Lines 1002 and 1004 illustrate the results of electrodes 102' that have been coated with a hydrophilic coating 102B', while lines 1006 and 1008 illustrate the results of electrodes 102 that have not been coated with a hydrophilic coating 102B'. As seen in FIG. 10, application of the hydrophilic coating does not result in a statistically significant increase in impedance. Further, FIG. 11A illustrates, in an exemplary manner, multi-variable chart 1100A showing the signal from the electrodes 102' with the hydrophilic coating 102B', while FIG. 11B illustrates a multi-variable chart 1100B of the resistance of coated and non-coated electrodes. As exemplified by these figures, the addition of the coating 102B' does not negatively impact signal performance of the electrode 102' to any significant degree.

The disclosed technology described herein can be further understood according to the following clauses:
Clause 1. An end effector comprising: a flexible insulative material extending along a plane; a framework disposed in the flexible insulative material; and a flexible circuit disposed in the flexible insulative material and spaced apart from the framework, the flexible circuit comprising: a central branch segment extending along a longitudinal axis of the plane; a plurality of outer branch segments extending along the plane a distance away from the longitudinal axis, at least some of the outer branch segments extending distal to the central branch segment thereby defining a recess in a distal end of the end effector proximate the longitudinal axis; and a plurality of electrodes disposed along the central branch and the plurality of outer branch segments.
Clause 2. The end effector of Clause 1, further comprising another flexible circuit disposed in the flexible insulative material and spaced apart from the framework, said another flexible circuit being spaced apart from the flexible circuit on an opposite side of the framework, said another flexible circuit comprising: a central branch segment extending along a longitudinal axis of the plane; a plurality of outer branch segments extending along the plane a distance away from the longitudinal axis, at least some of the outer branch segments extending distal to the central branch segment thereby defining a recess in a distal end of the end effector proximate the longitudinal axis; and a plurality of electrodes disposed along the central branch and the plurality of outer branch segments.
Clause 3. The end effector of Clause 1, the plurality of electrodes comprising: a first set of electrodes disposed on the central branch segment; and a second set of electrodes disposed along an adjacent branch of the plurality of outer branch segments, the first set of electrodes and the second set of electrodes being unaligned with each other in a direction transverse to the longitudinal axis.
Clause 4. The end effector of Clause 3, the plurality of outer branch segments comprising a first set of outer branch segments and a second set of outer branch segments, the first set of outer branch segments extending distal to the central branch segment and the second set of outer branch segments terminating at a location approximately aligned with the central branch segment in a direction transverse to the longitudinal axis.
Clause 5. The end effector of Clause 4, wherein electrodes disposed along the second set of outer branch segments align with electrodes disposed along the central branch segment.
Clause 6. The end effector of Clause 1, the end effector further defining one or more voids between each of the plurality of outer branch segments and the central branch segment.
Clause 7. The end effector of Clause 6, wherein, a sum of the width of the central branch segment and the plurality of outer branch segments in a direction transverse to the longitudinal axis is less than a threshold width at any given location along the end effector.
Clause 8. The end effector of Clause 1, the plurality of outer branch segments extending outwardly from the central branch segment at an oblique angle with respect to the central branch segment for at least a portion of the plurality of outer branch segments.
Clause 9. The end effector of Clause 8, wherein a proximal portion of the outer branch segments extend outwardly from the central branch segment at an oblique angle with respect to the longitudinal axis and a distal portion of the outer branch segments extend parallel to the central branch segment.
Clause 10. The end effector of Clause 1, the flexible circuit further comprising a plurality of traces extending along the flexible circuit and a substrate supporting each trace of the plurality of traces, the substrate and the plurality of traces defining a plurality of gaps between each of the plurality of traces.
Clause 11. The end effector of Clause 10, the substrate and at least some of the traces of the plurality of traces further defining strain relief loops.
Clause 12. The end effector of Clause 1, further comprising a position sensor extending along the plane.
Clause 13. The end effector of Clause 12, the position sensor comprising a recess configured to at least partially align with the recess in the distal end of the end effector.
Clause 14. The end effector of Clause 1, the end effector being coated with a lubricious coating.
Clause 15. The end effector of Clause 1, the plurality of electrodes each being coated with a hydrophilic coating.
Clause 16. The end effector of Clause 15, the hydrophilic coating comprising a hydrogel.
Clause 17. The end effector of Clause 1, the plurality of electrodes each being coated with an impedance reducing coating.
Clause 18. The end effector of Clause 17, the impedance reducing coating comprising at least one of poly(3,4-ethylenedioxythiophene):polystyrene sulfonate, iridium oxide, platinum-iridium or a combination thereof.
Clause 19. The end effector of Clause 17, a hydrophilic coating being disposed at least partially over the impedance reducing coating.
Clause 20. An end effector comprising: a flexible insulative material extending along a plane; a framework disposed in the flexible insulative material; and a first flexible circuit disposed in the flexible insulative material and spaced apart from the framework on one side of the framework; and a second flexible circuit disposed in the flexible insulative material and spaced apart from both the framework and the first flexible circuit, each of the first and second flexible circuits comprising: a central branch segment extending along a longitudinal axis of the plane; a first set of electrodes disposed on the central branch segment; a plurality of outer branch segments extending along the plane a distance away from the longitudinal axis; and a second set of electrodes disposed along adjacent outer branch segments, the first set of electrodes and the second set of electrodes being unaligned with each other in a direction transverse to the longitudinal axis.
Clause 21. The end effector of Clause 20, at least some of the respective outer branch segments of the first flexible circuit and the second flexible circuit extending distal to the respective central branch segment, thereby defining a recess in a distal end of the end effector.
Clause 22. The end effector of Clause 20, the plurality of outer branch segments of the first flexible circuit and the second flexible circuit respectively comprising a first set of outer branch segments and a second set of outer branch segments, the first set of outer branch segments extending distal to the respective central branch segment and the second set of outer branch segments terminating at a location approximately aligned with the respective central branch segment in a direction transverse to the longitudinal axis.
Clause 23. The end effector of Clause 22, wherein electrodes disposed along the second set of outer branch segments of the first flexible circuit align with electrodes disposed along the central branch segment of the first flexible circuit.
Clause 24. The end effector of Clause 20, the end effector further defining one or more voids between each of the plurality of outer branch segments of the first flexible circuit and the central branch segment of the first flexible circuit.
Clause 25. The end effector of Clause 24, wherein, a sum of the width of the central branch segment of the first flexible circuit and the plurality of outer branch segments of the first flexible circuit in a direction transverse to the longitudinal axis is less than a threshold width at any given location along the end effector.
Clause 26. The end effector of Clause 25, the plurality of outer branch segments of the first flexible circuit extending outwardly from the central branch segment of the first flexible circuit at an oblique angle with respect to the central branch segment of the first flexible circuit for at least a portion of the plurality of outer branch segments of the first flexible circuit.
Clause 27. The end effector of Clause 26, wherein a proximal portion of the outer branch segments of the first flexible circuit extend outwardly from the central branch segment of the first flexible circuit at an oblique angle with respect to the longitudinal axis and a distal portion of the outer branch segments of the first flexible circuit extend parallel to the central branch segment of the first flexible circuit.
Clause 28. The end effector of Clause 20, the first flexible circuit further comprising a plurality of traces extending along the first flexible circuit and a substrate supporting each trace of the plurality of traces, the substrate and the plurality of traces defining a plurality of gaps between each of the plurality of traces.
Clause 29. The end effector of Clause 28, the substrate and at least some of the traces of the plurality of traces further defining strain relief loops.
Clause 30. The end effector of Clause 21, further comprising a position sensor extending along the plane.
Clause 31. The end effector of Clause 30, the position sensor comprising a recess configured to at least partially align with the recess in the distal end of the end effector.
Clause 32. The end effector of Clause 20, the end effector being coated with a lubricious coating.
Clause 33. The end effector of Clause 20, the plurality of electrodes each being coated with a hydrophilic coating.
Clause 34. The end effector of Clause 33, the hydrophilic coating comprising a hydrogel.
Clause 35. The end effector of Clause 20, the plurality of electrodes each being coated with an impedance reducing coating.
Clause 36. The end effector of Clause 35, the impedance reducing coating comprising at least one of poly(3,4-ethylenedioxythiophene):polystyrene sulfonate, iridium oxide, or platinum-iridium.
Clause 37. The end effector of Clause 35, a hydrophilic coating being disposed at least partially over the impedance reducing coating.
Clause 38. A method of manufacturing an end effector for a medical probe, the method comprising: forming an electrode on a flexible circuit; coating the electrode with an impedance reducing coating; and coating the electrode with a hydrophilic coating.
Clause 39. The method of Clause 38, the hydrophilic coating comprising a hydrogel.
Clause 40. The method of Clause 38 further comprising coating the end effector, including the electrode, with the hydrophilic coating.
Clause 41. The method of Clause 38, the impedance reducing coating comprising at least one of poly(3,4-ethylenedioxythiophene):polystyrene sulfonate, iridium oxide, or platinum-iridium.
Clause 42. A method of manufacturing a flexible circuit for a medical probe, the method comprising: forming a substrate extending along a plane; disposing a conductive material along the substrate; and cutting through the conductive material and the substrate along a curve of the flexible substrate such that the flexible substrate and the conductive material form a plurality of individual branches.
Clause 43. The method of Clause 42, wherein disposing the conductive material along the substrate comprises disposing individual traces of the conductive material along the substrate.
Clause 44. The method of Clause 42, wherein disposing the conductive material along the substrate comprises disposing a single continuous portion of conductive material along the substrate.
Clause 45. The method of Clause 42 further comprising forming strain relief loops in at least one of the plurality of individual branches.
Clause 46. The method of Clause 42, wherein cutting through the conductive material and the substrate comprises cutting through the conductive material and the substrate with a laser.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. An end effector comprising:
a flexible insulative material extending along a plane;
a framework disposed in the flexible insulative material; and
a flexible circuit disposed in the flexible insulative material and spaced apart from the framework, the flexible circuit comprising:
a central branch segment extending along a longitudinal axis of the plane;
a plurality of outer branch segments extending along the plane a distance away from the longitudinal axis, at least some of the outer branch segments extending distal to the central branch segment thereby defining a recess in a distal end of the end effector proximate the longitudinal axis; and
a plurality of electrodes disposed along the central branch and the plurality of outer branch segments.

2. The end effector of Claim 1, further comprising another flexible circuit disposed in the flexible insulative material and spaced apart from the framework, said another flexible circuit being spaced apart from the flexible circuit on an opposite side of the framework, said another flexible circuit comprising:
a central branch segment extending along a longitudinal axis of the plane;
a plurality of outer branch segments extending along the plane a distance away from the longitudinal axis, at least some of the outer branch segments extending distal to the central branch segment thereby defining a recess in a distal end of the end effector proximate the longitudinal axis; and
a plurality of electrodes disposed along the central branch and the plurality of outer branch segments.

3. The end effector of Claim 1, the plurality of electrodes comprising:
a first set of electrodes disposed on the central branch segment; and
a second set of electrodes disposed along an adjacent branch of the plurality of outer branch segments, the first set of electrodes and the second set of electrodes being unaligned with each other in a direction transverse to the longitudinal axis.

4. The end effector of Claim 3, the plurality of outer branch segments comprising a first set of outer branch segments and a second set of outer branch segments, the first set of outer branch segments extending distal to the central branch segment and the second set of outer branch segments terminating at a location approximately aligned with the central branch segment in a direction transverse to the longitudinal axis.

5. The end effector of Claim 4, wherein electrodes disposed along the second set of outer branch segments align with electrodes disposed along the central branch segment.

6. The end effector of Claim 1, the end effector further defining one or more voids between each of the plurality of outer branch segments and the central branch segment.

7. The end effector of Claim 6, wherein, a sum of the width of the central branch segment and the plurality of outer branch segments in a direction transverse to the longitudinal axis is less than a threshold width at any given location along the end effector.

8. The end effector of Claim 1, the plurality of outer branch segments extending outwardly from the central branch segment at an oblique angle with respect to the central branch segment for at least a portion of the plurality of outer branch segments.

9. The end effector of Claim 8, wherein a proximal portion of the outer branch segments extend outwardly from the central branch segment at an oblique angle with respect to the longitudinal axis and a distal portion of the outer branch segments extend parallel to the central branch segment.

10. The end effector of Claim 1, the flexible circuit further comprising a plurality of traces extending along the flexible circuit and a substrate supporting each trace of the plurality of traces, the substrate and the plurality of traces defining a plurality of gaps between each of the plurality of traces.

11. An end effector comprising:
a flexible insulative material extending along a plane;
a framework disposed in the flexible insulative material; and
a first flexible circuit disposed in the flexible insulative material and spaced apart from the framework on one side of the framework; and
a second flexible circuit disposed in the flexible insulative material and spaced apart from both the framework and the first flexible circuit, each of the first and second flexible circuits comprising:
a central branch segment extending along a longitudinal axis of the plane;
a first set of electrodes disposed on the central branch segment;
a plurality of outer branch segments extending along the plane a distance away from the longitudinal axis; and
a second set of electrodes disposed along adjacent outer branch segments, the first set of electrodes and the second set of electrodes being unaligned with each other in a direction transverse to the longitudinal axis.

12. The end effector of Claim 11, at least some of the respective outer branch segments of the first flexible circuit and the second flexible circuit extending distal to the respective central branch segment, thereby defining a recess in a distal end of the end effector.

13. The end effector of Claim 11, the plurality of outer branch segments of the first flexible circuit and the second flexible circuit respectively comprising a first set of outer branch segments and a second set of outer branch segments, the first set of outer branch segments extending distal to the respective central branch segment and the second set of outer branch segments terminating at a location approximately aligned with the respective central branch segment in a direction transverse to the longitudinal axis, optionally wherein electrodes disposed along the second set of outer branch segments of the first flexible circuit align with electrodes disposed along the central branch segment of the first flexible circuit.

14. The end effector of Claim 11, the end effector further defining one or more voids between each of the plurality of outer branch segments of the first flexible circuit and the central branch segment of the first flexible circuit, preferably wherein, a sum of the width of the central branch segment of the first flexible circuit and the plurality of outer branch segments of the first flexible circuit in a direction transverse to the longitudinal axis is less than a threshold width at any given location along the end effector, more preferably the plurality of outer branch segments of the first flexible circuit extending outwardly from the central branch segment of the first flexible circuit at an oblique angle with respect to the central branch segment of the first flexible circuit for at least a portion of the plurality of outer branch segments of the first flexible circuit, still more preferably wherein a proximal portion of the outer branch segments of the first flexible circuit extend outwardly from the central branch segment of the first flexible circuit at an oblique angle with respect to the longitudinal axis and a distal portion of the outer branch segments of the first flexible circuit extend parallel to the central branch segment of the first flexible circuit.

15. The end effector of Claim 11, the first flexible circuit further comprising a plurality of traces extending along the first flexible circuit and a substrate supporting each trace of the plurality of traces, the substrate and the plurality of traces defining a plurality of gaps between each of the plurality of traces, optionally the substrate and at least some of the traces of the plurality of traces further defining strain relief loops.
